# EUROPEAN PATENT APPLICATION

(11) **EP 4 085 897 A1**
(43) Date of publication of application: **09.11.2022**
(21) Application number: 20941697.3
(22) Date of filing: 11.11.2020
(51) Int. Cl.: A61K 9/06, A61K 47/02, A61K 47/36, A61K 47/34, A61K 48/00, A61P 15/00, A61P 15/02, A61P 31/22, A61P 35/00

(54) **VAGINAL GEL PREPARATION AND PREPARATION METHOD THEREFOR**

(30) Priority: 24.06.2020 CN 202010594395
(71) Applicant: Zhuhai Shu Tong Medical Technology Co., Ltd., Zhuhai, Guangdong 519000 (CN)
(72) Inventor: XIE, Hongxian, Zhuhai, Guangdong 519000 (CN)
(74) Representative: Bryers LLP
(86) International application number: PCT/CN2020/128051
(87) International publication number: WO 2021/258614

(57) **Abstract**

A vaginal gel preparation and a preparation method therefor, with the vaginal gel preparation comprising a carrier and a therapeutic gene; and the vaginal gel preparation further comprises a hydrophilic polymer material modified montmorillonite, and the carrier comprises a cationic polymer. The vaginal gel preparation can better stabilize the structure of nanoparticles, has a high transfection efficiency, a good treatment effect and a high safety, and can be applied locally through the vagina. The preparation process of the vaginal gel preparation is simple, and the preparation formula is flexible.

## Description

### TECHNICAL FIELD OF THE INVENTION

The present disclosure belongs to the field of gene therapy, specifically, relates to a vaginal gel preparation and a preparation method therefor.

### BACKGROUND OF THE INVENTION

Genome editing has shown promising success to treat genetic disorders. Nevertheless, one of the keys to successful gene therapy is the delivery of the genome engineering tools. Owing to the nanoscale sizes, low toxicity, long cycle time and excellent plasticity, polymer based nanoparticles (NPs) have achieved efficient delivery for gene therapy.

Based on specific therapeutic aims and pharmacokinetics, gene therapy can be performed systemically or locally. The vagina has several features that facilitate the delivery of therapeutic molecules, including DNA, RNA, and proteins. The vaginal route has an adequate blood supply, high contact area, and appropriate permeability for several substances, allowing systemic and local administration. It also avoids the hepatic first-pass effect and gastrointestinal fluids encountered with oral administration. In addition, some local diseases, such as lesions caused by papillomavirus (HPV) infection and even cervical cancer, can be treated by local vaginal administration. Compared with systemic administration, local vaginal administration has unique advantages in the treatment of reproductive tract infections. For example, the local use of genome editing tools to treat HPV infections could improve treatment outcomes and minimize off-target side effects. However, some great obstacles remain to be overcome before the technology can be translated into clinical medicine. A major difficulty in developing vaginal gene therapy drugs is that exogenous genetic material is easily degraded by local microorganisms and the passage of exogenous genetic material through vaginal and cervical mucus is inefficient. Therefore, protecting genes from being degraded and improving their mucus penetration ability are key issues in the development of vaginal gene delivery systems. In addition, in order to avoid flowing out of the open reproductive tract, the preparations need to be formulated in the form of suppositories, gels, tablets, vaginal rings, etc., to achieve the purpose of retention and long-term release in the reproductive tract. However, it is a great challenge to keep the structure of gene delivery nanoparticles stable in these preparations. The composition of vaginal and cervical mucus is complex, and if nanoparticles cannot maintain their structural stability in the preparations (such as decomposing into cationic polymers and genes), they are easily affected by proteins/enzymes, bacterial secretions, inorganic salts, etc. in the mucus after release and cannot self-assemble into a gene-cationic polymer nanoparticle structure with high transfection efficiency, thereby affecting gene transfection and subsequent therapeutic effects. Therefore, the development of preparations that can keep the nanoparticle structure stable is an important part of vaginal gene delivery systems.

### SUMMARY OF THE INVENTION

The present disclosure is designed to provide a vaginal gel preparation suitable for vaginal administration and capable of effectively delivering exogenous genes, and a preparation method therefor.

To achieve the above purpose, a technical solution employed by the present disclosure is:
An aspect of the present disclosure provides a vaginal gel preparation comprising a vector and a therapeutic gene, the vaginal gel preparation further comprises a hydrophilic polymer material-modified montmorillonite, and the vector comprises a cationic polymer.

Preferably, the hydrophilic polymer material-modified montmorillonite is obtained by modifying montmorillonite with one or more modifiers selected from the group consisting of chitosan, chitooligosaccharide, cellulose derivatives, starch derivatives, gelatin, shellac, tragacanth, gum arabic, pectin, xanthan gum, povidone, polyvinyl alcohol and polyoxyethylene.

Further preferably, the mass ratio of the hydrophilic polymer material to montmorillonite in the hydrophilic polymer material-modified montmorillonite is (0.1 to 1): 1, preferably (0.1 to 5): 1.

The unmodified montmorillonite raw material used in the present disclosure is preferably natural montmorillonite, which contains Al₂O₃ 16.54%, MgO 4.65%, and SiO₂ 50.95%, whose structural formula is (Al, Mg)₂ [SiO₁₀] (OH)₂·nH₂O, which belongs to monoclinic crystal system.

Preferably, the hydrophilic polymer material-modified montmorillonite further contains sodium salt.

Further preferably, the sodium salt is one or more selected from the group consisting of sodium bicarbonate, disodium hydrogen phosphate, disodium hydrogen citrate and sodium acetate.

Further preferably, the mass ratio of the sodium salt to montmorillonite is (0.001 to 0.05): 1, preferably (0.005 to 0.05): 1.

Preferably, the mass ratio of the cationic polymer to the hydrophilic polymer material-modified montmorillonite is 1: (1 to 1000), further preferably 1: (1 to 100), more preferably 1: (1 to 40).

Preferably, the preparation method of the vaginal gel preparation comprises steps of preparing nanoparticles by using the vector and the therapeutic gene, and mixing the nanoparticles, the hydrophilic polymer material-modified montmorillonite and water.

Further preferably, the mass ratio of the vector to the therapeutic gene in the nanoparticles is (10 to 150): 1, further preferably (10 to 100): 1, more preferably (10 to 80): 1.

Further preferably, the particle size of the nanoparticles is in the range of 10 to 1000 nm, preferably 200 to 400 nm.

Further preferably, the surface of the nanoparticles is charged.

Preferably, the cationic polymer is one or more selected from the group consisting of poly(β-amino ester) (PBAE), polylysine (PLL), polyethyleneimine (PEI), and poly(amidoamine) (PAMAM).

Further preferably, the poly(β-amino ester) is prepared from 1,4-butanediol diacrylate monomer, 5-amino-1-pentanol monomer and 1-(3-aminopropyl)-4-methylpiperazine monomer.

Further preferably, the general structure of the poly(β-amino ester) is 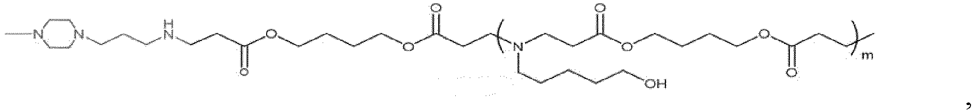 wherein m is a number between 5 and 200, preferably a number between 15 and 30.

Further preferably, the number-average molar mass of poly(β-amino ester) is in the range of 1500 to 60000, preferably 5000 to 8000.

Further preferably, the process for the preparation of the poly(β-amino ester)is: firstly reacting1,4-butanediol diacrylate and 5-amino-1-pentanol at 80 to 100 °C for 30 to 40 h, and post-treating to give an intermediate, then reacting the intermediate with 1-(3-aminopropyl)-4-methylpiperazine at 10 to 40 °C for 20 to 30 h, and post-treating to give the poly(β-amino ester).

More preferably, the molar ratio of 1,4-butanediol diacrylate to 5-amino-1-pentanol is 1: (0.8 to 1.2), and the molar ratio of the intermediate to 1-(3-aminopropyl)-4-methylpiperazine is 1: (4 to 6).

Preferably, the therapeutic gene is one or more selected from the group consisting of plasmids, DNA and RNA.

Further preferably, the therapeutic gene is a plasmid composed of DNA that targets the PERV-Pol gene and can be transcribed into sgRNA, and SpCas9.

More preferably, the sequence of the DNA that can be transcribed into sgRNA is shown in SEQ ID NO. 1 and/or SEQ ID NO. 2.

More preferably, the SpCas9 is SpCas9-HF1 and/or eSpCas9.

According to a specific and preferred implementation, the therapeutic gene is SpCas9-sgRNA-1 and/or SpCas9-sgRNA-3, wherein the sequence of the SpCas9-sgRNA-1 is shown in SEQ ID NO. 3, and the sequence of the SpCas9-sgRNA-1 is shown in SEQ ID NO. 4.

Preferably, the pH of the vaginal gel preparation is 4 to 8, more preferably 4.5 to 5.5.

In the present disclosure, the vaginal gel preparation contains nanoparticles.

The vaginal gel preparation in the present disclosure also contains an acidic buffer, and the acidic buffer is one or more selected from the group consisting of citric acid buffer, acetic acid buffer, and phosphoric acid buffer.

Further preferably, the pH of the acidic buffer is 4 to 6.

In the present disclosure, the concentration and amount of the acidic buffer and the amount of water can be adjusted according to the required viscosity of the gel preparation.

A second aspect of the present disclosure provides a vaginal gel preparation comprising a matrix and nanoparticles, the matrix contains a hydrophilic polymer material-modified montmorillonite, and the nanoparticles contain a cationic polymer and a therapeutic gene.

The hydrophilic polymer material-modified montmorillonite, the cationic polymer and the therapeutic gene in this solution are the same as the raw materials in the vaginal gel preparation provided in the first aspect of the present disclosure, and will not be repeated here.

According to a specific and prefered implementation, the hydrophilic polymer material-modified montmorillonite is obtained by modifying montmorillonite with one or more modifiers selected from the group consisting of chitosan, chitooligosaccharide, chitosan derivatives, cellulose derivatives, starch derivatives, gelatin, shellac, tragacanth, gum arabic, pectin, xanthan gum, povidone, polyvinyl alcohol and polyoxyethylene; the cationic polymer is poly(β-amino ester), and its general structure is , wherein m is a number between 5 and 200; the therapeutic gene is a plasmid formed by SpCas9-HF1 and/or eSpCas9, and DNA that can be transcribed into sgRNA-1 and/or DNA that can be transcribed into sgRNA-3, wherein, the sequence of the DNA that can be transcribed into sgRNA-1 is TTCGAATGGAGAGATCCAGG, and the sequence of the DNA that can be transcribed into sgRNA-3 is GGTGACCCTCCTCCAGTACG.

A third aspect of the present disclosure provides a preparation method for the vaginal gel preparation, comprising the steps of:
forming nanoparticles with the vector and the therapeutic gene in an acidic buffer, to give a nanoparticle-containing mixture;
directly mixing the nanoparticle-containing mixture with the hydrophilic polymer material-modified montmorillonite to prepare the vaginal gel preparation, or, dispersing the hydrophilic polymer material-modified montmorillonite in water and/or an acidic buffer, and then mixing with the nanoparticle-containing mixture.

Preferably, the preparation method further comprises the step of preparing the hydrophilic polymer material-modified montmorillonite: adding montmorillonite and hydrophilic polymer material into water, selectively adding sodium salt, mixing and stirring, and then adjusting pH to 4 to 6, stirring and standing, drying and pulverizing the upper layer of colloidal liquid to give the hydrophilic polymer material-modified montmorillonite.

Further preferably, the temperature of the mixing and stirring is 50 to 90 °C, and the time is 1 to 10 h.

Further preferably, after adjusting the pH, stirring at a constant temperature of 50 to 90 °C for 0.5 to 2 h, then cooling, adding water and stirring evenly, and then standing.

Further preferably, using an acid to adjust the pH, the acid is one or more selected form the group consisting of phosphoric acid, citric acid and acetic acid.

According to a specific and preferred implementation, the preparation method for the hydrophilic polymer material-modified montmorillonite is as follows: adding montmorillonite and sodium salt into water and mixing evenly, adding the hydrophilic polymer material and stirring, heating up to 50 to 90 °C under sealing and stirring, keeping for 1 to 10 h, adjusting pH to 4 to 6 with acid, stirring at a constant temperature for 0.5 to 2 h, cooling to room temperature, adding water and stirring evenly, and standing for 30 to 60 min, and drying and pulverizing the upper layer of colloidal liquid to give the hydrophilic polymer material-modified montmorillonite.

Preferably, the specific method of the step (3) is: mixing the hydrophilic polymer material-modified montmorillonite with water, then adding an acidic buffer, adding the nanoparticles, and stirring evenly.

The fourth aspect of the present disclosure is to provide a vaginal delivery method of therapeutic gene, which comprises firstly preparing the therapeutic gene and a vector into nanoparticles, and then mixing the nanoparticles with a hydrophilic polymer material-modified montmorillonite to prepare into a gel preparation, and then applying the gel preparation into the vagina.

The fifth aspect of the present disclosure is to provide an administration method, wherein the vaginal gel preparation is administered locally in the vagina.

The sixth aspect of the present disclosure is to provide use of the vaginal gel preparation in gene therapy for vaginal/cervical diseases.

Specifically, the diseases comprise cervical lesions associated with HPV infection, cervical cancer or herpes simplex virus (HSV) infection and the like.

The vaginal gel preparation of the present disclosure only needs to be stored at about 18 to 25 °C, and the preparation can be used by oneself, which provides convenience for patients and doctors.

Due to the use of the above technical solutions, the present disclosure has the following advantages over the prior art:
The vaginal gel preparation can better stabilize the structure of nanoparticles, has a high transfection efficiency, a good treatment effect and a high safety, and can be applied locally through the vagina.

The preparation process of the vaginal gel preparation of the present disclosure is simple, and the preparation formula is flexible.

### BRIEF DESCRIPTION OF THE DRAWINGS

Figure 1 shows the characterization results of PBAE polymer and PBAE-plasmid multimer NPs;
Figure 2 is the scanning electron microscope image of each matrix or gel;
Figure 3 is a graph showing the results of the release of NPs in the NPs-mMMT gel;
Figure 4 is *in vivo* imaging diagrams of mice;
Figure 5 is a comparison of the transfection efficiency results of gel preparations prepared with different cationic polymers;
Figure 6 is a comparison of the transfection efficiency results of the gel preparations prepared by mMMT or HTT;
Figure 7 is the positive proportion of GFP+ cells in vaginal smear;
Figure 8 is the experimental result of Embodiment 4;
Figure 9 is the experimental result of Embodiment 5;
Figure 10 shows the results of immunochemical detection of inflammatory cytokines IFN-γ and TNF-α.

### DETAILED DESCRIPTION OF EXEMPLARY EMBODIMENTS

In the following, the present disclosure is further explained combining with embodiments shown in the accompanying drawings. However, the present disclosure is not limited to the following embodiments. The implementation conditions adopted in the embodiments can be further adjusted according to different requirements of specific use, and the unspecified implementation conditions are the conventional conditions in the industry.

The main raw materials used in the following embodiments are as follows:
1,4-butanediol diacrylate (BDD) and 5-amino-1-pentano (AP) were purchased from TCI (Shanghai, China).
1-(3-aminopropyl)-4-methylpiperazine (AMP) was purchased from Alfa Aesar (L04876, USA).
Polyethyleneimine 25 kD (PEI 25 kD) (Cat, 28968) was purchased from Poly science (400 valley Road, Warrington).
Polylysine was purchased from Aladdin Reagent.
PAMAM was purchased from Chenyuan (Weihai, Shandong).
pMAX-GFP (CAS: 8603168) was purchased from Bio Vector NTCC (Beijing, China).
Plasmids encoding Cas9 and sgRNA (CRISPR/Cas9 plasmid) were purchased from Addgene (#58778).

Three sgRNAs directing/targeting the PERV-pol gene (Accession No. AJ279056.1) were designed according to the method of Mali et al. (Mali P, Yang L, Esvelt KM, Aach J, Guell M, DiCarlo JE, et al. RNA-guided human genome engineering via Cas9. Science. 2013;339(6121):823-6), and synthesized by GENEWIZ (Jiangsu, China). The specific sequence of the double-stranded oligonucleotide tag (Tsai SQ, Zheng Z, Nguyen NT, Liebers M, Topkar VV, Thapar V, et al. GUIDE-seq enables genome-wide profiling of off-target cleavage by CRISPR-Cas nucleases. Nature biotechnology. 2015;33(2):187-97) was: forward, 5' G^{∗}T^{∗}TTAATTGAGTTGTCATATGTTAATAACGGT^{∗}A^{∗}T 3'; reverse, 5' A^{∗}T^{∗}ACCGTTATTAACATATGACAACTCAATTAA^{∗}A^{∗}C 3' (^{∗} indicates phosphorylation). The method of preparing the "oligonucleotide tag" was: dissolving the two oligonucleotides in STE buffer (10 mM Tris pH 8.0, 50 mM NaCl, 1 mM EDTA) at a concentration of 2 OD/100 µL and mixing equimolarly, incubating at 94 °C for 5 min, and cooling to room temperature. All sgRNAs used in the present disclosure are shown in Table 1.

### Embodiment 1: Preparation of Gels

### I. Synthesis of PBAE Polymers

BDD, AP, and AMP were prepared into PBAE polymers as follows: First, BDD (stabilized with p-hydroxyanisole MEHQ) and AP were mixed in a molar ratio of 1: 1, and stirred on a magnetic stirring plate at 90 °C under N₂ atmosphere for 36 h. After the above reaction, they were diluted with dimethylformamide (DMF), precipitated, and then washed with cold diethyl ether. After removal of ether by vacuum drying, the intermediate product (i-PBAE) was dried at room temperature. Then, i-PBAE was dissolved in anhydrous DMF, and reacted with 5-fold molar amount of AMP at room temperature with stirring for 24 h, and then the reaction product was precipitated in cold anhydrous diethyl ether. Finally, the final polymer (PBAE) was washed three times with diethyl ether, dried under vacuum for 48 h, and stored at room temperature for later use.

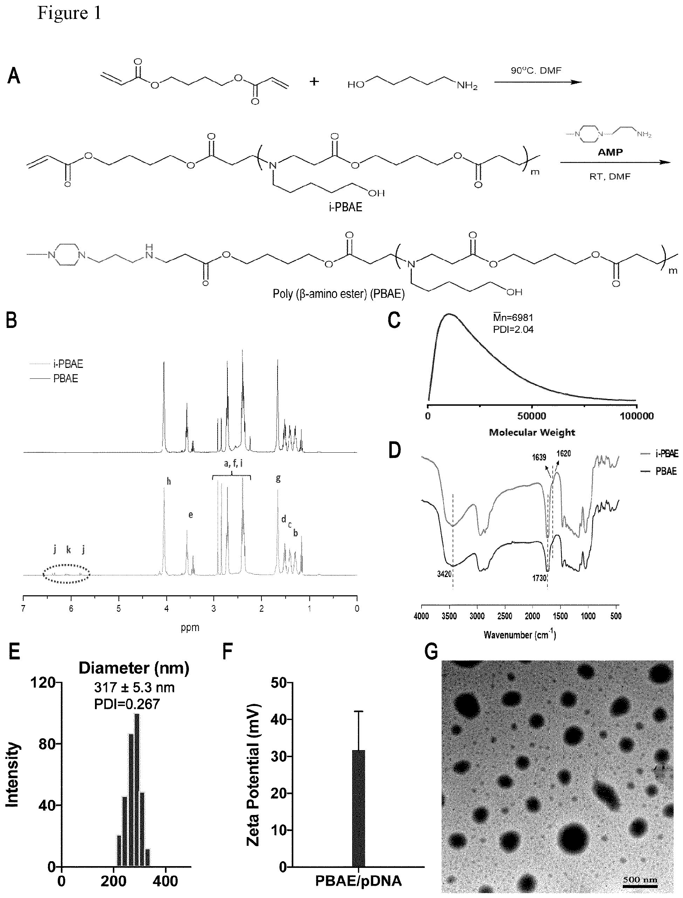

The synthetic equation of the PBAE polymer is shown in Figure 1A.

The structures of i-PBAE and PBAE were characterized by ¹H-NMR spectroscopy (Bruker AVANCE III 400MHz NMR spectrometer, solvent: CDCl₃), and the spectra are shown in Figure 1B, the peak with chemical shift value of 4.062 ppm belongs to the hydrogen signal peak of -COOCH₂- on the BDD unit of PABE and i-PABE, the 1.32 - 1.53 ppm region is the hydrogen signal peak of methylene -CH₂- on AP, the 2.42, 2.65 and 2.85 ppm peaks are the hydrogen signal peaks of -N(CH₂)₂-, -NCH₂CH₂OCO and -NCH₂CH₂OCO-, the 5.80 - 6.40 ppm region of the i-PABE spectrum is the hydrogen signal peak of the terminal group of allylic, which was not detected from the synthesized PABE spectrum, indicating a successful reaction between the C-C double bond and AMP.

The number-average molecular weight (Mn) of PBAE prepared in this embodiment was 6980 Da, which was determined by gel permeation chromatography (GPC, Waters-2410 system), Waters 2414 refractive index detector (mobile phase: DMF, standard: narrow-dispersion polystyrene), the sample was dissolved in dimethylformamide (DMF) with a concentration of 0.3 wt%, and the test results are shown in Figure 1C.

The structures of i-PBAE and PBAE were characterized by Fourier transform infrared spectroscopy (FTIR, PerkinElmer Spectrum Two, 32 repeated scan settings), the spectra are shown in Figure ID, the peak at 1730 cm⁻¹ is specifically from the fatty ester -CH₂-COO-CH₂- on the BDD chain of i-PABE and PBAE. In addition, the broad peak between 3200 - 3500 cm⁻¹ proves the presence of -OH on the main chain, while the unique 1639 and 1620 cm⁻¹ peaks of i-PBAE are hydrocarbon stretching vibration peaks of C-C double bond, which also disappeared in the PBAE spectrum. The above results were consistent with the results of ¹H-NMR, indicating that PBAE was successfully synthesized.

### II. Preparation and Characterization of PBAE-plasmid Multimeric NPs

To prepare PBAE-plasmid multimeric NPs, PBAE was dissolved in citrate buffer with a pH value of 5.0 and dissolved by ultrasonication to give a polymer solution (with a concentration of 20 mg/mL). The polymer solution and plasmid were uniformly mixed at the weight ratio of PBAE to plasmid of 75: 1, vortexed, and incubated at room temperature for 30 min to obtain PBAE-plasmid multimeric NPs.

The characterization of PBAE-plasmid multimeric NPs was carried out by dynamic light scattering (DLS, Zetaplus, Brookhaven, USA) and transmission electron microscopy (Hitachi HT7700, Japan), and the test results are shown in Figures IE and IF, and the scanning electron microscopy (TEM) detection results are shown in Figure 1G.

It was found that the optimal ratio of PBAE to pDNA was 75: 1, and at this time, as can be seen from Figures 1E and 1F, the average particle size was 317.1 ± 5.3 nm, the PDI was 0.267 (Figure 1E) and the surface charge was 34.9 ± 6.5 mV (Figure IF).

The vector materials PBAE and pDNA were compressed through electrostatic interactions, so the NPs were compressed and densely charged. By transmission electron microscopy (TEM), we observed that the morphology of the composite NPs is shown in Figure 1G. Electron micrographs show that the composite NPs formed by PBAE are all spherical particles with a size distribution around 240 nm and have excellent dispersion properties, which are smaller than the results of the DLS test. This phenomenon is caused by the dehydration and shrinkage of the multipolymeric NPs.

For PEI-plasmid multimeric NPs, polylysine-plasmid multimeric NPs, PAMAM-plasmid multimeric NPs, etc., it is only necessary to use PEI, polylysine, PAMAM, etc. instead of PBAE to mix with the plasmid, wherein the plasmid DNA and 25 kda liner PEI were mixed at a mass ratio of 10: 1, and the mass ratio of the remaining cationic polymer to plasmid was also 75: 1.

### III. Preparation of Vaginal Gel Preparations

Preparation of NPs-HTT gel: 66.65 mg of hectorite (HTT) was mixed with 1 mL of water in a magnetic stirrer at 1000 - 1500 rpm, and after dispersion for 20-30 min, 0.11 mL of 10 × citric acid buffer (citric acid: 1 mol/L, sodium citrate: 1 mol/L, pH: 4.7) was added, and then 1 mL of PBAE-plasmid multimeric NPs (containing 250 µg of plasmid DNA and 100 µg of oligonucleotide tags) was added, and the system was mixed well, and the pH of the gel was 5.0.

Preparation of NPs-mMMT Gel:
1. Preparation of Modified Montmorillonite mMMT
   100 mg of medicinal natural montmorillonite was mixed with 1 mg of disodium hydrogen citrate and 20 mg of water, and stirred evenly. 10 mg of methyl cellulose modifier was added and stirred. The temperature was slowly raised to 70 °C under sealing and stirring, and kept for 5 h. The system was adjusted to pH of 5 with citric acid, stirred at constant temperature for 1 h, and cooled to room temperature, then 250 mg of water was added, and stirred evenly. The system stood for 40 min, and the upper colloidal liquid was taken out and dried and pulverized to give mMMT.
2. Preparation of NPs-mMMT Gel
   60 mg of mMMT was mixed with 1 mL of water in a magnetic stirrer at 1000 - 1500 rpm and dispersed for 20 - 30 min. 0.11 mL of 10 × citric acid buffer (citric acid: 1 mol/L, sodium citrate: 1 mol/L, pH: 4.7) was added, and then 1 mL of PBAE-plasmid multimeric NPs (containing 250 µg of plasmid DNA and 100 µg of oligonucleotide tags) was added, and the system was mixed well, and the pH of the gel was 5.0.

Preparation of NPs-F127 gel: it was the same as the preparation method of the above two gels, except that HTT or mMMT was replaced with a thermosensitive gel Pluronic F127, and the pH of this gel was 5.0.

Other cationic polymer gels only need to use corresponding polymer-plasmid composite nanoparticles to replace PBAE-plasmid composite nanoparticles, for example, use PEI-plasmid composite nanoparticles, polylysine-plasmid composite nanoparticles, PAMAM-plasmid composite nanoparticles, etc. to replace PBAE-plasmid composite nanoparticles, hereinafter referred to as NPs or composite nanoparticles.

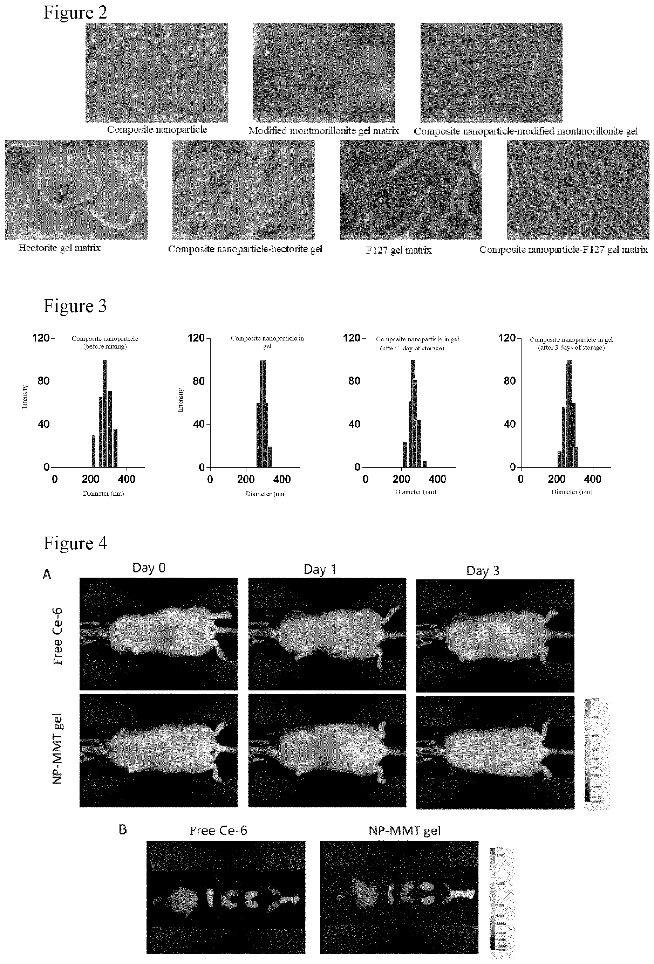

The PBAE-plasmid composite nanoparticles (i.e., the composite nanoparticles in Figure 2), the modified montmorillonite gel matrix (i.e., the matrix prepared without adding 1 mL of PBAE-plasmid multimeric NPs when the NPs-mMMT gel was prepared above), the NPs-mMMT gel (i.e., composite nanoparticle-modified montmorillonite gel in Figure 2), the hectorite gel matrix (i.e., the matrix prepared without adding 1 mL of PBAE-plasmid multimeric NPs when the NPs-HTT gel was prepared above), the NPs-HTT gel (i.e., the composite nanoparticle-hectorite gel in Figure 2), the F127 gel matrix (i.e., the matrix prepared without adding 1 mL of PBAE-plasmid multimeric NPs when the NPs-F127 gel was prepared above), and the NPs-F127 gel (i.e., the composite nanoparticle-F127 gel matrix in Figure 2) were observed at 2kV with a scanning electron microscope (SEM, Hitachi SU8010, Japan), and the test results are shown in Figure 2. Before the test, the samples were fixed on silicon wafers in the form of coatings, and then sprayed with metal, and to observe the stability of the formulations, the gels were stored at room temperature for 3 days, and then observed by SEM.

The NPs-mMMT gel was diluted with 4 × volumes of citrate buffer and filtered with a 0.45 µm filter to test the release of NPs from the gel, and the solution was tested with DLS to measure the diameters of the NPs released from the gel, and the sizes of the NPs were measured in the same way after different storage times (1 day and 3 days) at room temperature, and the test results are shown in Figure 3.

It can be seen from Figure 2 and Figure 3 that the NPs in the mMMT gel did not change, which could maintain the stability of the NP structure, and could be directly released in the form of nanoparticles from the gel matrix after dilution, and had good storage stability, and the released nanoparticles remained unchanged in particle diameter after three days of storage at room temperature. However, in the HTT gel and the thermosensitive gel Pluronic F127, the NPs were greatly affected by the matrix and could not maintain the nanoparticle structure.

### Embodiment 2: In vivo Experiments in Mice

The vaginal retention capacity of NPs-mMMT gel in a Kunming mouse model was evaluated by *in vivo* imaging techniques. The fluorescent dye Ce-6 was mixed into the composite nanoparticles at a mass ratio of 1% of the total mass of the composite nanoparticles to give Ce-6-labeled NPs, and then Ce-6-labeled PBAE-plasmid composite nanoparticle gel (NPs-mMMT gel) was prepared according to the solution of Embodiment 1. Then, the free fluorescent dye Ce-6 and Ce-6-labeled PBAE-plasmid composite nanoparticle gels were put into the mouse vagina respectively, the test results are shown in Figure 4, and the signal of the free fluorescent dye (Ce-6) was disappeared within in one day. For mMMT gels, strong fluorescence can be seen on Day 3. These results indicate that mMMT gel has a good vaginal retention ability and can improve the action time of gene drugs in the vagina.

### Embodiment 3: Experiments in Pigs

### (I) Comparison of Transfection Effects of Different Cationic Polymers

To evaluate the transfection efficiency of our vaginal gel preparation, in the preparation process of the PBAE-plasmid composite nanoparticles, PEI-plasmid composite nanoparticles, and PAMAM-plasmid composite nanoparticles in Embodiment 1, when preparing the composite nanoparticles according to the method of Embodiment 2, the plasmid (reporter gene) was a green fluorescent protein plasmid (GFP).

Large mammal pigs were selected as the experimental subjects, because the genome of pigs is very close to that of humans, and the pigs were one-month-old ordinary domestic pigs.

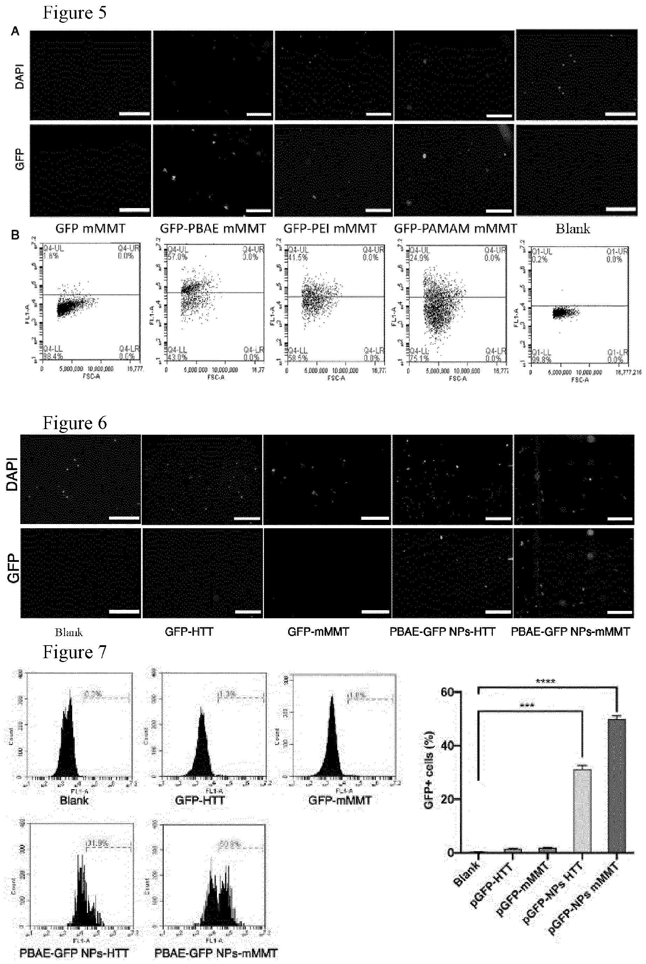

During administration, each pig was given one of GFP-labeled PBAE-plasmid composite nanoparticles, PEI-plasmid composite nanoparticles, and PAMAM-plasmid composite nanoparticles, and the dosage was 2 mL, and the vaginal gel was spread evenly over the vaginal surface, cervical surface and posterior vaginal fornix of the pig. Dosing was done on Day 1 and Day 4, followed by collection of cervical cells with a brush (similar to Pap smear) on Day 7, the sampled cervical cells were stored in cervical cell preservation solution and collected by centrifugation at 715 g for 5 min. Then, the cells were resuspended in the same volume of PBS and directly analyzed after staining with DAPI, and the number of cells in a certain volume was counted with a cytometer to calculate the total number of cells collected. For each group, approximately 2.5 × 10⁴ cells were taken for detection by fluorescence microscopy and flow cytometry, then the transfection efficiency of each group was detected by fluorescence microscopy (Leica DMI8) and flow cytometry (C6, BD, USA), and the test results are shown in Figure 5. The cells in the blank group had almost no green fluorescence, and the GFP-mMMT without cationic polymer had almost no GFP expression, while the GFP-PBAE mMMT, GFP-PEI mMMT, and GFP-PAMAM mMMT groups showed obvious GFP expression, which were 57.0 %, 41.5% and 24.9%, respectively. Obviously, when using mMMT + cationic material PBAE to prepare vaginal gel, a higher proportion of GFP-positive cells can be obtained, thereby achieving efficient intravaginal gene delivery.

### (II) Comparison of Transfection Effects of mMMT and HTT

In the preparation process of the PBAE-plasmid multimeric NPs of Embodiment 1, the green fluorescent protein (GFP) was mixed into the composite nanoparticles at a mass ratio of 1% of the total mass of the composite nanoparticles according to the method of Embodiment 2, and then PBAE-GFP NPs-mMMT and PBAE-GFP NPs-HTT were prepared using mMMT and HTT as matrixes according to the solution of Embodiment 1, respectively.

The preparation of GFP-HTT and GFP-mMMT was basically the same as the preparation of PBAE-GFP NPs-mMMT and PBAE-GFP NPs-HTT described above, except that PBAE was omitted.

Then, the transfection efficiency was tested according to the same method as the first section of this embodiment, and the test results are shown in Figure 6.

Meanwhile, the cervix and the organs (ovaries, urethra, vagina and uterus) were collected, rinsed with PBS to remove any adhering fat or any other extra tissue, then fixed in 4% paraformaldehyde and cut into 3 - 5 µm thick sections for H & E staining and IHC staining, and the test results are shown in Figure 7, at the same time, three groups were repeated and the proportion of GFP+ cells in the vaginal smear was counted, and the result was 0.30% ± 0.05% in the blank group; 1.41% ± 0.28% in the GFP-HTT group; 1.80% ± 0.24% in the GFP-mMMT group; 25.79% ± 1.56% in the PBAE-GFP NPs-HTT group; and 51.12% ± 1.23% in the PBAE-GFP NPs-mMMT group, respectively.

Clearly, a much higher proportion of GFP-positive cells was obtained when using mMMT material to prepare vaginal gels (p value < 0.0001). Meanwhile, PBAE-free delivery showed low transfection efficiency, suggesting that the cationic polymer PBAE is necessary as a gene delivery vector. In summary, the data indicate that our PBAE-GFP NPs-mMMT gel is more efficient than PBAE-GFP NP-HTT gel for *in vivo* intravaginal DNA delivery.

### Embodiment 4: Vaginal Virus Removal with Gel System

Porcine endogenous retroviruses (PERVs) were integrated into the genome of all pigs and released as infective particles. Under certain conditions, they can infect human cells and share similarities with human sexually transmitted viruses. Therefore, this study selected PERVs as therapeutic targets to test the ability of the gel system to clear viral integrated fragments in large mammal pigs.

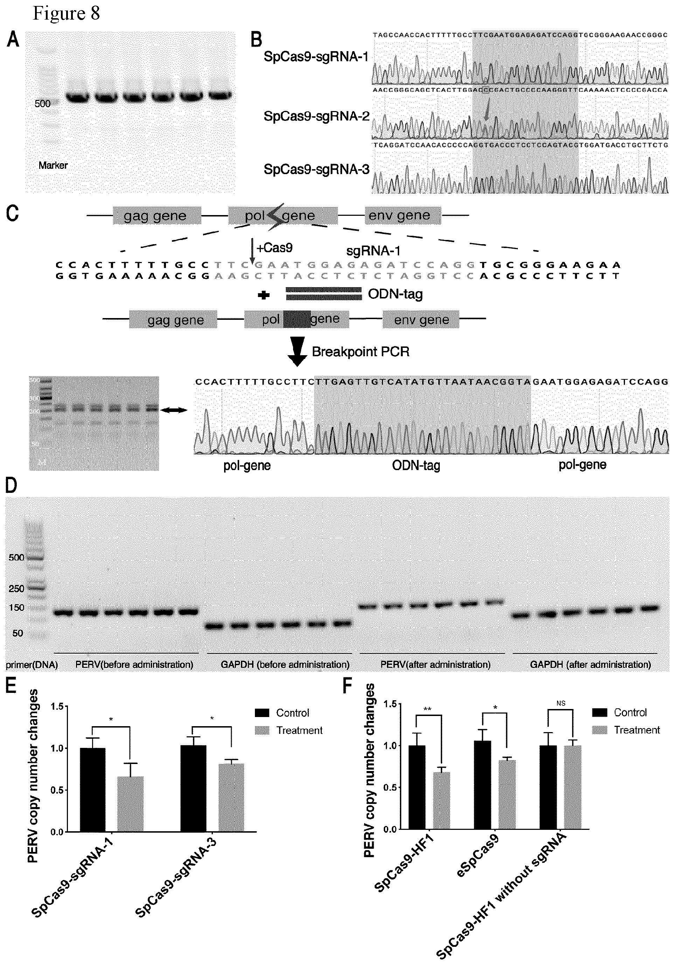

PERV is a retrovirus that can integrate its DNA into the pig genome to form a provirus. Its genome is mainly composed of three genes, namely PERV-gag, pol and env, which can be replicated together with the host genome. Previous studies confirmed that the copy number of pol gene in tissues is higher than that of gag and env genes, so sgRNAs targeting PERV-pol gene was designed. According to the website of Zhang's lab (Doench JG, Fusi N, Sullender M, Hegde M, Vaimberg EW, Donovan KF, et al. Optimized sgRNA design to maximize activity and minimize off-target effects of CRISPR-Cas9. Nature biotechnology. 2016;34(2): 184-91), three sgRNAs with the highest scores were selected and SpCas9-sgRNA plasmids (SpCas9-sgRNA-1, SpCas9-sgRNA-2, SpCas9-sgRNA-3) were constructed. Then a vaginal mMMT gel containing the corresponding PBAE plasmid NPs was prepared (the preparation method was the same as that of Embodiment 1). Our previous experiments showed that the editing efficiency of the CRISPR/Cas9 system can be affected if the target is mutated. Therefore, before animal experiments, Sanger sequencing of PERV DNA was routinely performed to confirm whether there were mutations in the PERV sequence (see Figure 8A for the test results). The results showed that the target DNA of sgRNA-2 has a base mutation, which may affect the editing efficiency of the CRISPR/Cas9 system (see Figure 8B for the test results). Therefore, sgRNA-1 and sgRNA-3 were selected for further experiments.

During administration, each pig was given 2 mL of PBAE-CRISPR/Cas9 plasmid mMMT gel (containing 250 µg of CRISPR/Cas9 gene editing system plasmid) (that is, the NPs-mMMT gel prepared in Embodiment 1), which was evenly distributed on the vaginal surface, cervical surface and posterior vaginal fornix of 1-month-old pigs. Dosing was done on Day 1 and Day 4, and cells were harvested on day 7. The brush was then rotated three times around the female pigs' cervical to obtain cells (this is very similar to the Pap smear collection method in human patients). The cervical cells were taken and placed in cervical cell preservation solution, and centrifuged at 715 g for 5 min. Then, the cells were resuspended in the same volume of PBS, and the number of cells in a certain volume was counted with a cytometer to calculate the total number of cells collected. About 2.5×10⁴ cells were taken from each group for analysis.

### DNA Extraction and PCR

DNA was extracted with DNeasy Blood & Tissue Kit (69506 Qiagen, Germany). PCR reactions were performed with Q5 Hot-Start High-Fidelity 2X Master Mix (M0494S, NEW ENGLAND BioLabs, USA). The primers used are listed in Table 1. qPCR was performed on a Bio-Rad CFX96 instrument using PowerUpSYBRGreen Master Mix (A25742, Applied Biosystems, USA). The experiment was carried out with three biological replicates, and the porcine GAPDH gene was used as the internal reference gene, and the 2-ΔΔCT method was used to calculate the relative expression changes of PERV copy number. The primers used are listed in Table 1.

**Table 1 PCR and qPCR Primers**

| Name | Forward | Reverse |
|---|---|---|
| PERV-PCR | aatactcccctgctaccggt | aatctgggccttcttagcgg |
| PERV-RTPCR | gagactacatcccactagccaac | tagggcttcgtcaaagatggtc |
| GAPDH-pig | ccgcgatctaatgttctctttc | ttcactccgaccttcaccat |

It was demonstrated that the PBAE NPs-based mMMT gel could efficiently introduce the CRISPR/Cas9 system into the porcine vaginal epithelium, target PERV at this site, and significantly reduce the viral copy number. The PBAE-SpCas9/sgRNA-1 NPs-mMMT vaginal gel successfully reduced the viral copy number of PERV after applying it to the porcine vagina (Figure 8E).

If SpCas9 cleaves PERV DNA in the vagina *in vivo,* oligo inserts into the cleavage site. PCR reactions of the insertion site genome and oligo DNA (one primer on the oligo and the other primer on the genomic locus close to the target) yielded the correct size of about 200 bp (see Figure 8C for the test results). As expected, Sanger-Sequencings demonstrated that the SpCas9-PERV sgRNA plasmid efficiently cleaved the PERV DNA sequence in porcine cervical epithelial cells *in vivo,* and the oligo was correctly inserted (Figure 8C). Further qPCR also showed a reduction in PERV copy number in the PBAE-SpCas9 NPs gel-treated group. Compared with before treatment, the PERV copy number in the sgRNA-1 group decreased by 34%, and the PERV copy number in the sgRNA-3 group decreased by 17%, indicating that the cleavage efficiency of sgRNA-1 was better than that of sgRNA-3 (Figure 8E).

To reduce off-target sites without affecting genome editing efficiency, the specificity of the SpCas9 nuclease was increased by two variants: eSpCas9 and SpCas9-HF1. Therefore, in this study, the effectiveness of eSpCas9 and SpCas9-HF1 as well as sgRNA-1 in mMMT gels was further tested. Compared with the control group, the PERV copy number was reduced by about 42% in the SpCas9-HF1 group and 34% in the eSpCas9 group, indicating that the SpCas9-HF1-based vaginal gel was more effective (P<0.05, Figure 8F). Since plasmid delivery can trigger an inflammatory response that affects PBAE copy number, the PBAE-spcas9-HF1 (without sgRNA) NPs-mMMT gel of the experimental group was added as another control group to observe this phenomenon. It was found that the PERV copy number in the SpCas9-HF1 group without sgRNA treatment did not change compared with before treatment (Figure 8F), indicating that plasmid delivery did not cause an immune response that affected the PERV copy number.

In summary, these data demonstrate that our *in vivo* DNA delivery based on PBAE-Cas9 NPs-mMMT vaginal gel has achieved efficient local genome editing.

### Embodiment 5: Safety Evaluation of Gels

After the pigs were euthanized, their cervix, ovaries, urethra, vagina and uterus were isolated and fixed (4% paraformaldehyde). Paraffin-embedded sections (5 mm) were subjected to IHC staining according to Proteintech protocol (http://www.ptgcn.com/support/protocols).

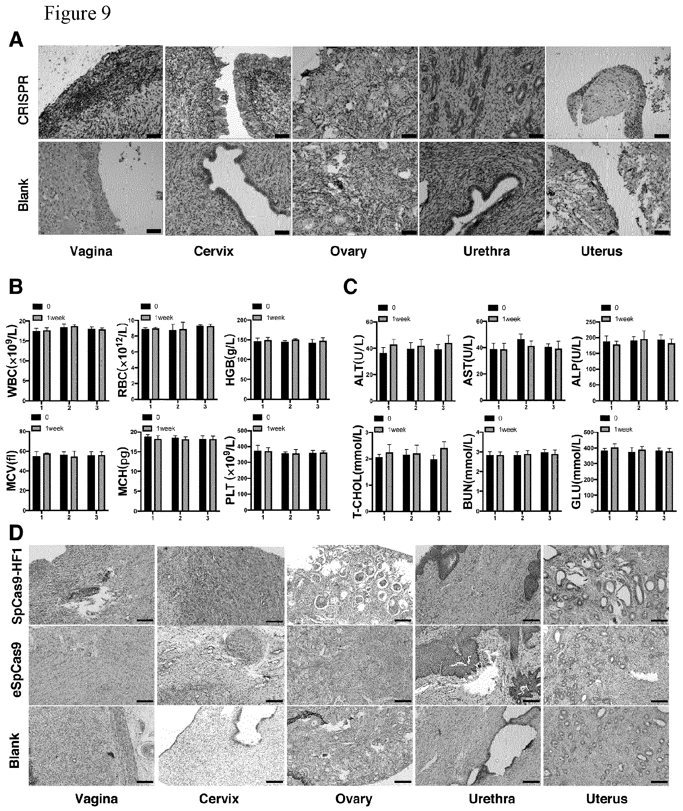

The biodistribution of the delivery system was first assessed. Because the CRISPR/Cas9 plasmid in our system contains a Flag tag, the Flag is expressed when the plasmid is delivered to a location in a pig organ and successfully transfected. The expression of the Flag tag in different organs such as vagina, urethra, cervix, ovary and uterus was stained by immunohistochemistry (IHC). It can be seen that only the vaginal and cervical tissues show the Flag, with the vagina being the strongest. The tags were not expressed in other organ tissues, indicating that our system works locally in the vagina and cervix and does not spread to nearby organs (Figure 9A). Next, blood routine and blood biochemical tests were performed on NPs-mMMT gel-treated pigs to evaluate their toxicity. Blood tests reflect the normal metabolism of multiple systems throughout the body, including the hematopoietic and immune systems. Alanine aminotransferase (ALT), aspartate aminotransferase (AST) and alkaline phosphatase (ALP) in blood biochemical indicators are related to liver function, while alanine aminotransferase (BUN) is an important indicator of kidney. Normal mMMT gel was administered on Day 1 and Day 4, blood and serum were collected to perform blood tests and blood biochemical tests on the day before treatment and on Day 7 of treatment. There was no significant difference in blood routine indicators such as WBC, RBC and the like compared with before administration (P<0.05, Figure 9B). Blood biochemical tests yielded similar results (Figure 9C). Further hematoxylin-eosin staining (H&E) was performed on the cervix, ovary, urethra, vagina and uterus to evaluate the toxicity of the drug, and the results showed no abnormal morphology, pathological edema, inflammatory necrosis and apoptotic bodies (Figure 9D).

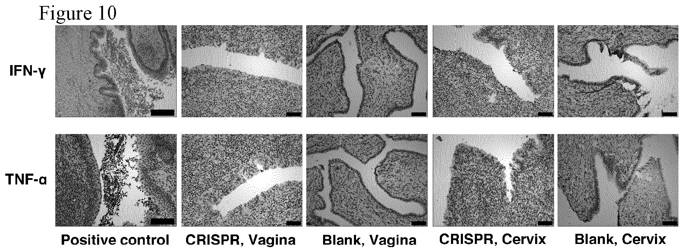

Immunochemical detection of inflammatory cytokines IFN-γ and TNF-α in vagina, cervix, and urethral cavity in the PBAE-SpCas9/sgRNA NPs-mMMT-treated and control groups was also performed to confirm the results. No obvious inflammatory cytokine staining was observed (Figure 10). The data suggest that our drug delivery formulation is relatively safe, with local vaginal biodistribution and low toxicity, which also facilitates the future application of the gel system in clinical trials.

All quantitative data from the above statistical analysis are the mean ± SD from at least three replicates. Statistical analysis was performed using one-way ANOVA analysis and calculated by post-hoc test and T-test with GraphPad Prism software (GraphPad Prism 8), where P<0.05 was considered a significant difference.

The embodiments described above are only for illustrating the technical concepts and features of the present disclosure, and are intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

## Claims

1. A vaginal gel preparation, comprising a vector and a therapeutic gene, **characterized in that**, the vaginal gel preparation further comprises a hydrophilic polymer material-modified montmorillonite, and the vector comprises a cationic polymer.

2. The vaginal gel preparation according to claim 1, **characterized in that**, the hydrophilic polymer material-modified montmorillonite is obtained by modifying montmorillonite with one or more modifiers selected from the group consisting of chitosan, chitooligosaccharide, chitosan derivatives, cellulose derivatives, starch derivatives, gelatin, shellac, tragacanth, gum arabic, pectin, xanthan gum, povidone, polyvinyl alcohol and polyoxyethylene.

3. The vaginal gel preparation according to claim 1 or 2, **characterized in that**, the mass ratio of the hydrophilic polymer material to montmorillonite in the hydrophilic polymer material-modified montmorillonite is (0.1 to 1): 1.

4. The vaginal gel preparation according to claim 1 or 2, **characterized in that**, the hydrophilic polymer material-modified montmorillonite further contains sodium salt.

5. The vaginal gel preparation according to claim 4, **characterized in that**, the sodium salt is one or more selected from the group consisting of sodium bicarbonate, disodium hydrogen phosphate, disodium hydrogen citrate and sodium acetate.

6. The vaginal gel preparation according to claim 1, **characterized in that**, the mass ratio of the cationic polymer to the hydrophilic polymer material-modified montmorillonite is 1: (1 to 1000).

7. The vaginal gel preparation according to claim 1, **characterized in that**, a preparation method of the vaginal gel preparation comprises steps of preparing nanoparticles by using the cationic polymer and the therapeutic gene, and mixing the nanoparticles, the hydrophilic polymer material-modified montmorillonite and water.

8. The vaginal gel preparation according to claim 1 or 7, **characterized in that**, the mass ratio of the vector to the therapeutic gene is (10 to 150): 1.

9. The vaginal gel preparation according to claim 7, **characterized in that**, the surface of the nanoparticles is charged.

10. The vaginal gel preparation according to claim 1 or 7, **characterized in that**, the cationic polymer is one or more selected from the group consisting of poly(β-amino ester), polylysine, polyethyleneimine, and poly(amidoamine).

11. The vaginal gel preparation according to claim 1 or 7, **characterized in that**, the therapeutic gene is one or more selected from the group consisting of plasmids, DNA and RNA.

12. The vaginal gel preparation according to claim 1, **characterized in that**, the pH of the vaginal gel preparation is 4.0 to 8.0.

13. The vaginal gel preparation according to claim 1, **characterized in that**, the vaginal gel preparation contains nanoparticles.

14. A vaginal gel preparation, **characterized in that**, it comprises a matrix and nanoparticles, the matrix contains a hydrophilic polymer material-modified montmorillonite, the nanoparticles contain a cationic polymer and a therapeutic gene, and the hydrophilic polymer material-modified montmorillonite is obtained by modifying montmorillonite with one or more modifiers selected from the group consisting of chitosan, chitooligosaccharide, chitosan derivatives, cellulose derivatives, starch derivatives, gelatin, shellac, tragacanth, gum arabic, pectin, xanthan gum, povidone, polyvinyl alcohol and polyoxyethylene; the cationic polymer is poly(β-amino ester), and its general structure is 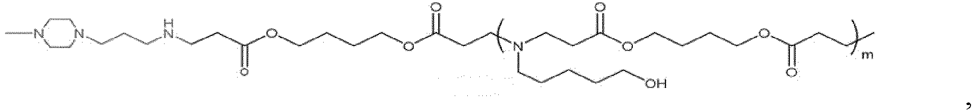 wherein m is a number between 5 and 200; the therapeutic gene is a plasmid formed by SpCas9-HF1 and/or eSpCas9, and DNA that can be transcribed into sgRNA-1 and/or DNA that can be transcribed into sgRNA-3, wherein, the sequence of the DNA that can be transcribed into sgRNA-1 is TTCGAATGGAGAGATCCAGG, and the sequence of the DNA that can be transcribed into sgRNA-3 is GGTGACCCTCCTCCAGTACG.

15. A preparation method for the vaginal gel preparation according to any one of claims 1 to 14, **characterized in that**, it comprises the following steps:
forming nanoparticles with the vector and the therapeutic gene in an acidic buffer, to give a nanoparticle-containing mixture;
directly mixing the nanoparticle-containing mixture with the hydrophilic polymer material-modified montmorillonite to prepare the vaginal gel preparation, or, dispersing the hydrophilic polymer material-modified montmorillonite in water and/or an acidic buffer, and then mixing with the nanoparticle-containing mixture.

16. The preparation method for vaginal gel preparation according to claim 15, **characterized in that**, the preparation method further comprises the step of preparing the hydrophilic polymer material-modified montmorillonite: adding montmorillonite and hydrophilic polymer material into water, selectively adding sodium salt, mixing and stirring, and then adjusting pH to 4 to 6, stirring and standing, drying and pulverizing the upper layer of colloidal liquid to give the hydrophilic polymer material-modified montmorillonite.

17. The preparation method for vaginal gel preparation according to claim 16, **characterized in that**, the temperature of the mixing and stirring is 50 to 90 °C, and the time is 1 to 10 h; after adjusting the pH, stirring at a constant temperature of 50 to 90 °C for 0.5 to 2 h, then cooling, adding water and stirring evenly, and then standing.

18. A vaginal delivery method of a therapeutic gene, **characterized in that**, the vaginal gel preparation according to any one of claims 1 to 14 is administered into vagina.

19. An administration method, **characterized in that**, the vaginal gel preparation according to any one of claims 1 to 14 is administered in the form of partial vaginal delivery.

20. Use of the vaginal gel preparation according to any one of claims 1 to 14 in gene therapy for vaginal and/or cervical diseases.
